# EUROPÄISCHE PATENTSCHRIFT

(11) **EP 1 989 175 B1**
(45) Veröffentlichungstag und Bekanntmachung des Hinweises auf die Patenterteilung: **19.09.2012**
(21) Anmeldenummer: 07722859.1
(22) Anmeldetag: 20.02.2007
(51) Int. Cl.: C07C 231/08, C07C 233/03

(54) **VERFAHREN ZUR HERSTELLUNG VON 1-FORMAMIDO-3,5-DIMETHYLADAMANTAN**
METHOD FOR PRODUCING 1-FORMAMIDO-3,5-DIMETHYLADAMANTANE
PROCÉDÉ POUR PRODUIRE LE COMPOSÉ 1-FORMAMIDO-3,5-DIMÉTHYLADAMANTANE

(30) Priorität: 01.03.2006 DE 102006009279
(43) Veröffentlichungstag der Anmeldung: 12.11.2008
(73) Patentinhaber: Merz Pharma GmbH & Co. KGaA, 60318 Frankfurt (DE)
(72) Erfinder: SCHREINER, Peter, R., 35415 Pohlheim (DE); FOKIN, Andrey, A., 35398 Giessen (DE); WANKA, Lukas, 61206 Wöllstadt (DE); WOLFE, Derek, M., Athens GA 30605 (US)
(74) Vertreter: Ricker, Mathias
(86) Internationale Anmeldenummer: PCT/EP2007/001433
(87) Internationale Veröffentlichungsnummer: WO 2007/101536

(56) Entgegenhaltungen:
- WO-A-2006/010362
- US-A- 5 117 056
- DATABASE CAPLUS CHEMICAL ABSTRACTS SERVICE, COLUMBUS, OHIO, US; 19. Oktober 2005 (2005-10-19), XIAOJUN ZHANG: "Preparation of a Substituted Symmetry Adamantane Amine Derivatives" XP002441200 Database accession no. AN:1117324 -& CN 1 566 075 A (XIAOJUN ZHANG) 19. Januar 2005 (2005-01-19)
- YU. N. KLIMOCHKIN ET AL.: "Synthesis of Alkoxycarbonylaminoadamantanes and Acetylaminoadamantanes in Nitric Acid" BULLETIN OF THE ACADEMY OF SCIENCES OF THE USSR, Bd. 37, Nr. 4, 1988, Seiten 757-759, XP002441198 USSR
- EDWARD T. ROE ET AL.: "Fatty Acid Amides. I. Preparation of Amides of Oleic and the 9,10-Dihydroxystearic Acids", JOURNAL OF THE AMERICAN CHEMICAL SOCIETY, vol. 71, no. 6, 1949, pages 2215-2218,

## Beschreibung

Gegenstand der Erfindung ist ein Verfahren zur Herstellung von 1-Formamido-3,5-dimethyladamantan, welches ein wichtiges Zwischenprodukt zur Herstellung des gegen die Alzheimer-Erkrankung verwendeten 1-Amino-3,5-dimethyladamantans ist, das unter dem Wirkstoffnamen Memantine bekannt ist. Es wird in Europa (und zahlreichen außereuropäischen Ländern) unter den Marken A-xura^{®} und Ebixa^{®} und in den USA unter Namenda® vertrieben.

Die Darstellung des 1-Formamido-3,5-dimethyladamantans aus 1-Brom-3,5-Dimethyladamantan und Formamid ist an sich bereits bekannt (CN 155 60 75).

Das 1-Amino-3,5-dimethyladamantan ist als Substanz bereits seit langem aus der DE 2 318 461 A1 bekannt. Seine Verwendung zur Prävention und Behandlung der zerebralen Ischämie ist in der EP 0 392 059 B1 beschrieben. Die Synthese des zu seiner Gewinnung verwendeten 1-Formamido-3,5-dimethyladamantans erfolgt demnach bisher durch eine Halogenierung des 1,3-Dimethyladamantans mit anschließender Formamidierung. Danach wird das 1-Formamido-3,5-dimethyladamantan mit verdünnter Salzsäure zum Amin hydrolysiert.

Allerdings erfordert dieses Syntheseverfahren drei Syntheseschritte, um vom 1,3-Dimethyladamantan zum Memantine zu gelangen: Halogenierung, Formamidierung und saure Hydrolyse. Dabei ist der Einsatz von giftigem, elementarem Chlor oder Brom im Überschuss erforderlich; das verursacht zusätzliche Kosten bei der Entsorgung und kann zu unerwünschten Nebenprodukten führen.

Es stellte sich deshalb die Aufgabe, ein Verfahren zur Formamidierung des 1,3-Dimethyladamantans bereitzustellen, welches einfacher ist und mit weniger giftigen oder teuren Reagenzien durchgeführt werden kann.

In der internationalen Patentanmeldung WO2006/010362 A1 ist bereits ein Verfahren zur Herstellung von 3,5-substituierten 1-Aminoadamantanderivaten beschrieben worden, bei dem ein 1,3-disubstituiertes Adamantanderivat in HNO₃ und H₂SO₄ suspendiert und nach Zugabe von Oleum mit einem Nitril umgesetzt wird. Demgegenüber erfolgt im erfindungsgemäßen Verfahren die Herstellung von 1-Formamido-3,5-dimethyladamantan in nur zwei Reaktionsschritten durch direkte Formamidierung von 1,3-Dimethyladamantan mit Formamid in konzentrierten Säuren, wobei als konzentrierte Säuren 30-70%-ige, insbesondere 65%-ige Salpetersäure und 90-100%-ige, vor allem aber 95-98%-ige Schwefelsäure eingestzt werden Die Umsetzung erfolgt im Allgemeinen bei -40 °C bis 50 °C, bevorzugt jedoch bei 0 °C. Bei dem erfindungsgemäßen Verfahren werden meistens Ausbeuten von 40-95-% erzielt.

Dieses Verfahren zeichnet sich nicht nur durch seine Halogenfreiheit aus, sondern weist auch ein günstigeres Verunreinigungsprofil auf, da es in hohen Ausbeuten und mit einem nur geringen Anteil an Nebenprodukten verläuft und dabei sogar Verunreinigungen in den Ausgangsmaterialien toleriert. Gemäß GC-MS konnten bei der Formamidierung als Nebenprodukte nur unumgesetztes Edukt sowie 3,5-Dimethyladamantan-1-ol nachgewiesen werden.

Sogar eine in situ-Herstellung des Ausgangsmaterials aus preisgünstigen Vorläufern ist möglich, so dass in das gewünschte Endprodukt in einem "Eintopf"-Verfahren erhältlich ist. Dabei wird das mit Formamid umzusetzende 1,3-Dimethyladamantan in situ aus einem Kohlenwasserstoff der Summenformel C₁₂H₂₀ durch kationische Umlagerung unter vergleichbaren Reaktionsbedingungen, wie sei auch für die Formamidierung angewendet werden, erhalten. Die Vorläufer erhält man beispielsweise durch Perhydrierung von Acenaphthen, Acenaphthalen oder Methylcyclopentadien-Dimer. Anschließend wird im gleichen Reaktionsgefäß die Formamidierung des entstandenen Dimethyladamantans unter sauren Bedingungen durchgeführt.

Die Behandlung von perhydriertem Methylcyclopentadien-Dimer mit konzentrierten Säuren ermöglicht die Darstellung des Ausgangsmaterials 1,3-Dimethyladamantan in situ vor der Formamidierung nach folgender Reaktion:

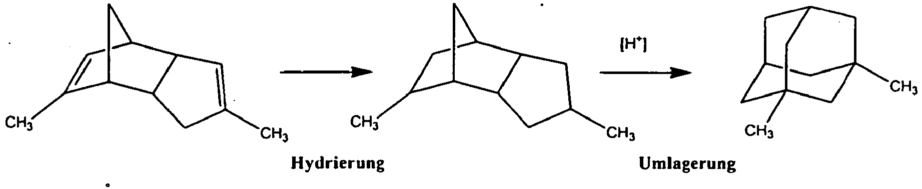

Das in der internationalen Anmeldung WO 2006/020362 A1 beschriebene Verfahren baut auf der Erzeugung von Carbokationen in konzentrierten Säuren auf, die durch ein Nukleophil, wie beispielsweise ein Nitril, abgefangen werden und bei wässriger Aufarbeitung in die korrespondierenden 1-Amidoadamantan-Derivate nach folgender Reaktion übergehen:

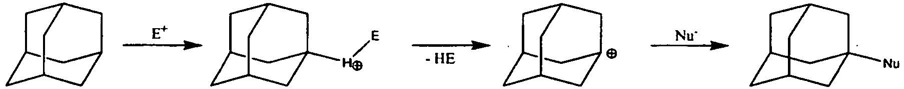

Demgegenüber gestattet das erfindungsgemäße Verfahren zur Synthese von 1-Formamido-3,5-dimethyladamantan mildere Reaktionsbedingungen unter völliger Vermeidung der Anwendung von Oleum oder 100%-iger Salpetersäure. Weiterhin wird als Nucleophil Formamid verwendet, wobei als Reaktionsprodukt 1-Formamido-3,5-dimethyladamantan entsteht, das gegenüber anderen Amiden unter deutlich milderen Bedingungen hydrolysiert werden kann. So wird beispielsweise 1-Acetamido-3,5-dimethyladamantan durch mehrstündiges Erhitzen von NaOH in wässriger oder alkoholischer Base oder konzentrierter Salzsäure (36-37%-ig) zum freien 1-Amino-3,5-dimethyladamantan gespalten, wohingegen die Spaltung des Formamids bereits mit verdünnter Salzsäure innerhalb zwei Stunden bei 100 °C gelingt.

Die Erfindung wird durch die folgenden Beispiele näher erläutert:

### Beispiel 1 - Darstellung von 1-Formamido-3,5-dimethyladamantan

Zu 6,572 g (40mmol) 1,3-Dimethyladamantan werden bei 0 °C nacheinander 4 mL 65%-ige, technische Salpetersäure und darauf innerhalb von drei Stunden 50 mL 98%-ige, technische Schwefelsäure gegeben. Man rührt über Nach bei 0 °C und gießt die Mischung bei 0 °C auf 100 mL Formamid in einen Rundkolben, der mit einem Trockenrohr versehen wird. Man rührt diese Mischung 30 Minuten bei 0 °C und 90 Minuten bei Raumtemperatur weiter und gibt 200 mL Dichlormethan und 200 mL Wasser hinzu. Nach Phasentrennung wird die organische Phase mit Wasser und 2%-iger NaHCO₃-Lösung gewaschen, mit Na₂SO₄ getrocknet und am Rotationsverdampfer von Lösungsmitteln befreit. Das verbleibende Öl wird chromatographisch gereinigt wird (SiO₂, CHCl₃/Aceton (20 : 1), R_{f} = 0.39). Man erhält 7.41 g (89,3%) des Formamids als nahezu farblosen Feststoff.
¹H-NMR (CDCl₃, TMS, 400.13 MHz): δ = 0.87 ppm, s, 6 H; 1.15 ppm, s, 2 H; 1.2 - 1.35 ppm, m, 4 H; 1.35 - 1.55 ppm, m, 4 H; 1.65 - 1.78 ppm, m, 2 H; 2.10 - 2.27 ppm, m 1H; 5.90 und 7.21 ppm; jew. br. s, 1H; 8.02, 8.20 und 8.27 ppm, jew. s. 1H.
¹³C-NMR (CDCl₃, TMS, 100.61 MHz): δ = 29.46, 30.01, 32.00, 40.21, 41.85, 47.63, 49.94, 51.83, 160.15/162.24 ppm.
MS: m/z = 207 (M⁺), 192, 150, 136, 106, 91, 79.

### Beispiel 2 -- Hydrolyse des 1-Formamide-3,5-dimethyladamantans zu 1-Amino-3,5-dimethyladamantan Hydrochlorid (Memantine)

0,02 mmol 1-Formamido-3,5-dimethyladamantan (4,14 g) werden in 100 mL 15%-iger Salzsäure 24 Stunden zum Rückfluss erhitzt. Nach dem Abkühlen wird der Niederschlag abfiltriert, in Methanol gelöst und durch Zugeben von Essigsäureethylester gefällt. (Ausbeute 80 %)

## Patentansprüche

1. Verfahren zur Herstellung von 1-Formamido-3,5-dimethyladamantan durch direkte Formamidierung von 1,3-Dimethyladamantan, **dadurch gekennzeichnet, dass** man das 1,3-Dimethyladamantan mit Formamid in konzentrierten Säuren, aber unter Vermeidung von SO₃-haltiger Schwefelsäure oder 100%-iger Salpetersäure, umsetzt, wobei als konzentrierte Säuren 30-70 %-ige Salpetersäure und 90-100 %-ige Schwefelsäure eingesetzt werden.

2. Verfahren nach Anspruch 1, **dadurch gekennzeichnet, dass** man das 1,3-Dimethyladamantan vor der direkten Formamidierung aus einem Kohlenwasserstoff-Vorläufer der Summenformel C₁₂H₂₀ durch kationische Umlagerung in einer getrennten Reaktion oder in situ herstellt.

3. Verfahren nach den Ansprüchen 1 und 2, **dadurch gekennzeichnet, dass** man das 1-Formamido-3,5-dimethyladamantan durch Hydrolyse in das 1-Amino-3,5-dimethyladamantan umwandelt.

4. Verfahren nach Anspruch 3, **dadurch gekennzeichnet, dass** die Hydrolyse mit wässriger Salzsäure erfolgt.

## Claims

1. Method for producing 1-formamido-3,5-dimethyladamantane by direct formamide formation of 1,3-dimethyladamantane, **characterized in that** said 1,3-dimethyladamantane is reacted with formamide in concentrated acids while avoiding SO₃-containing sulfuric acid or 100 % nitric acid, wherein 30 - 70 % nitric acid and 90 - 100 % sulfuric acid are used as concentrated acids.

2. Method according to claim 1, **characterized in that** 1,3-dimethyladamantane is made from a hydrocarbon precursor of the elemental formula C₁₂H₂₀ by cationic rearrangement in a separated reaction or *in situ* prior to the direct formamide formation.

3. Method according to claims 1 and 2, **characterized in that** 1-formamido-3,5-dimethyladamantane is converted into 1-amino-3,5-dimethyladamantane by hydrolysis.

4. Method according to claim 3, **characterized in that** said hydrolysis is performed using aqueous hydrochloric acid.

## Revendications

1. Procédé de production de 1-formamido-3,5-diméthyladamantane par formamidation directe de 1,3-diméthyladamantane, **caractérisé en ce que** ledit 1,3-diméthyladamantane est mis à réagir avec du formamide dans des acides concentrés en évitant l'acide sulfurique contenant du SO₃ ou l'acide nitrique à 100%, dans lequel de l'acide nitrique à 30-70% et de l'acide sulfurique à 90-100% sont utilisés en tant qu'acides concentrés.

2. Procédé selon la revendication 1, **caractérisé en ce que** le 1,3-diméthyladamantane est préparé à partir d'un précurseur hydrocarboné de formule élémentaire C₁₂H₂₀ par réarrangement cationique dans une réaction distincte ou *in situ* avant la formamidation directe.

3. Procédé selon les revendications 1 et 2, **caractérisé en ce que** le 1-formamido-3,5-diméthyladamantane est converti en 1-amino-3,5-diméthyladamantane par hydrolyse.

4. Procédé selon la revendication 3, **caractérisé en ce que** ladite hydrolyse est réalisée en utilisant de l'acide chlorhydrique aqueux.
